# EUROPEAN PATENT SPECIFICATION

(11) **EP 2 507 261 B1**
(45) Date of publication and mention of the grant of the patent: **21.12.2016**
(21) Application number: 10832439.3
(22) Date of filing: 30.11.2010
(51) Int. Cl.: C07K 14/78, A61L 27/22, C07K 14/50, A61K 38/18, A61P 17/02, C07K 14/65, A61K 38/39, C07K 14/485

(54) **FIBRONECTIN: GROWTH FACTOR CHIMERAS**
FIBRONECTIN: WACHSTUMSFAKTOREN-CHIMÄREN
CHIMÈRES DE FIBRONECTINE/FACTEUR DE CROISSANCE

(30) Priority: 30.11.2009 US 627647; 03.06.2010 US 793386
(43) Date of publication of application: 10.10.2012
(73) Proprietor: Factor Therapeutics Limited, Brisbane, QLD (AU)
(72) Inventor: UPTON, Zee, Indooroopilly Queensland 4068 (AU); VAN LONKHUYZEN, Derek, Morayfield Queensland 4506 (AU)
(74) Representative: Dzieglewska, Hanna Eva
(86) International application number: PCT/AU2010/001613
(87) International publication number: WO 2011/063477

(56) References cited:
- EP-A1- 0 795 606
- WO-A1-2004/069871
- WO-A2-2008/157483
- JP-A- 2008 125 460
- US-A- 5 302 701
- US-A1- 2009 209 730
- VAN LONKHUYZEN D ET AL: "Chimeric vitronectin:insulin-like growth factor proteins enhance cell growth and migration through co-activation of receptors", GROWTH FACTORS, HARWOOD ACADEMIC PUBLISHERS GMBH, XX, vol. 25, no. 5, 1 October 2007 (2007-10-01), pages 295-308, XP008105538, ISSN: 0897-7194, DOI: 10.1080/08977190701803752 [retrieved on 2007-12-07]
- NIMNI M E: "Polypeptide growth factors: targeted delivery systems", BIOMATERIALS, ELSEVIER SCIENCE PUBLISHERS BV., BARKING, GB, vol. 18, no. 18, 1 January 1997 (1997-01-01), pages 1201-1225, XP004086390, ISSN: 0142-9612, DOI: 10.1016/S0142-9612(97)00050-1
- PATENT ABSTRACTS OF JAPAN & JP 3 232 898 A (TAKARA SHUZO CO LTD) 16 October 1991
- ELEFTERIOU, F. ET AL.: 'Characterization of the Bovine Tenascin-X' THE JOURNAL OF BIOLOGICAL CHEMISTRY vol. 272, no. 36, 1997, pages 22866 - 22874, XP002532509
- NIES, D. E. ET AL.: 'The Complete cDNA Sequence of Human Hexabrachion (Tenascin) A Multidomain Protein Containing Unique Epidermal Growth Factor Repeats' THE JOURNAL OF BIOLOGICAL CHEMISTRY vol. 266, no. 5, 1991, pages 2818 - 2823, XP002146289
- JANG, J. -H. ET AL.: 'Engineering and expression of a recombinant fusion protein possessing fibroblast growth factor-2 and fibronectin fragment' BIOTECHNOLOGY LETTERS vol. 26, 2004, pages 1837 - 1840, XP019230813

## Description

### FIELD OF THE INVENTION

THIS INVENTION relates to protein complexes having respective domains that enable binding to and activation of both a growth factor receptor and an integrin receptor for fibronectin. In particular embodiments, this invention relates to chimeric proteins comprising growth factors such as insulin-like growth factor-I (IGF-I), insulin-like growth factor-II (IGF-II), epidermal growth factor (EGF), basic fibroblast growth factor (bFGF), or keratinocyte growth factor (EGF) receptor-binding domains and an integrin receptor-binding domain of fibronectin (FN). More particularly, this invention relates to protein complexes that stimulate cell migration and to compositions and methods that promote or induce cell migration and/or proliferation. These compositions and methods have use in wound healing, tissue engineering, cosmetic and therapeutic treatments such as skin replacement, and skin replenishment and treatment of burns where epithelial cell migration and/or proliferation is required. In other embodiments, the invention provides treatment provided by the present invention related to prevention or inhibition of cancer cell metastasis, particularly in relation to breast cancer.

### BACKGROUND OF THE INVENTION

A number of peptide growth factors involved in a broad range of cellular processes including hyperplasia, DNA synthesis, differentiation, cell cycle progression, and inhibition of apoptosis are known, and include the insulin-like growth factors (IGFs, *e.g.,* IGF-I and IGF-II) (Jones & Clemmons, 1995, Endocrine Rev. 16 3; Wood & Yee, 2000, J. Mammary Gland Biology and Neoplasia 5 1), EGF (Heldin et al., 1981, Science 4 1 122), bFGF (Taraboletti. et al., 1997, Cell Growth. Differ. 8 471), and KGF (Marchese et al., 1990, J. Cell Physiol. 144 326). These effects are mediated through binding to their cognate tyrosine-kinase linked cell surface receptors, the type 1 IGF receptor (IGF-IR), EGF receptor, bFGF receptor, and KGF receptor, respectively. The IGFs are also tightly regulated by a family of specific binding proteins, termed IGFBPs, whose primary role is to bind free IGFs and thereby moderate their half-life, specificity and activity (Clemmons, 1998, Mol. Cell. Endocrinol. 140 19).

Fibronectin is a high molecular mass adhesive glycoprotein found in all vertebrates. Fibronectin plays a role in cell adhesion, cell morphology and surface architecture. Its main function seems to be its involvement in cellular migration during development, tissue repair and wound healing, regulation of cell growth, and differentiation (Alitalo & Vaheri, 1982, Adv. Cancer Res. 37 1 1 1; Yamada, 1983, Annu. Rev. Biochem. 62 761; Hynes, 1985, Annu. Rev. Cell Biol. 1 67). Fibronectin polymorphism is due to alternative splicing patterns in three regions (ED-A, ED-B and IIICS) of the single fibronectin primary transcript (Petersen et al., 1983, Proc. Natl. Acad. Sci. USA 80 137; Schwarzbauer et al., 1983, Cell 35 421; Komblihtt et al., 1984, Nucleic Acids Res. 12 5853). The exact composition of fibronectin depends on the tissue type and/or cellular conditions. In humans, there are potentially 20 different forms of fibronectin, most arising from alternative splicing of some type 3 modules (Potts and Campbell, 1994, Curr. Opin. Cell Biol. 6 648). Expression of fibronectin splicing variants appears to be both developmentally regulated and tissue-specific.

Fibronectin has the ability to bind a number of extracellular molecules, including heparin, collagen and hyaluronic acid. Fibronectin organizes cell-cell interactions and cellular interaction with the extracellular matrix by binding to different components of the extracellular matrix and to membrane-bound fibronectin receptors (integrins) on cell surfaces.

However, the relative contributions of growth factors and fibronectin, and their respective domains, present in protein complexes, in terms of stimulating biological responses such as cell migration and/or proliferation, have remained elusive.

### SUMMARY OF THE INVENTION

The present inventors have discovered that protein complexes in the form of synthetic chimeras comprising growth factors such as IGF-I, IGF-II, EGF, bFGF, or KGF and FN stimulate cell migration and/or proliferation by binding and synergistically co-activating cognate growth factor receptors and FN-binding integrin receptors.

Therefore, the invention is broadly directed to isolated protein complexes that comprise a receptor-binding domain of a growth factor domain and at least a domain of fibronectin that is capable of binding an integrin receptor, wherein the isolated protein complex can co-activate the growth factor and integrin receptor to thereby elicit a biological response.

In a first aspect, the invention provides an isolated protein complex in the form of a synthetic chimeric protein comprising an amino acid sequence of:
(i) a growth factor, or at least a domain of a growth factor which is capable of binding a cognate growth factor receptor; and
(ii) one or more type-III domains of FN, wherein the one or more type-III domains of FN consist of amino acids 1266-1536 of the FN sequence set forth in SEQ ID NO: 1, or amino acids 1447-1536 of the FN sequence set forth in SEQ ID NO:1, or amino acids 1173-1536 of the FN sequence set forth in SEQ ID NO:1, which one or more type-III domains of FN comprise an integrin-binding domain.

Preferably, according to the aforementioned aspects the growth factor is IGF-I, IGF-II, EGF, bFGF, or KGF.

Preferably, the integrin-binding domain is an α₁ integrin-binding domain or an α₄ integrin-binding domain.

This aspect of the invention also contemplates an amino acid sequence of one or more additional fragments of fibronectin in the synthetic chimeric protein.

In a second aspect, the invention provides an isolated nucleic acid encoding the isolated protein complex of the first aspect.

In a third aspect, the invention provides a genetic construct comprising the isolated nucleic acid of the second aspect operably linked to one or more regulatory sequences in an expression vector.

Preferably, the genetic construct is an expression construct.

In a fourth aspect, the invention provides a host cell comprising the genetic construct of the third aspect.

In a fifth aspect, the invention provides a pharmaceutical composition comprising the isolated protein complex of the first aspect and a pharmaceutically-acceptable carrier, diluent or excipient.

This aspect of the invention may comprise the host cell of the fourth aspect, which cell expresses said synthetic protein(s).

In a sixth aspect, the invention provides an *in vitro* method of promoting cell migration including the step of using a synthetic protein to bind both a growth factor receptor and an integrin receptor.

Preferably, the growth factor receptor is IGF-IR, EGF receptor, bFGF receptor, or KGF receptor.

Preferably, the integrin receptor is an α₁ or an α₄ integrin.

Said synthetic protein is as according to the first aspect of the invention.

In a seventh aspect, the invention provides an isolated protein complex according to the first aspect of the invention, for use in wound or burn healing in an animal, preferably a human.

It will also be appreciated that the method of the seventh aspect may encompass prophylactic and therapeutic methods of treatment.

In an eighth aspect, the invention provides a biomaterial that comprises the isolated protein complex of the first aspect.

In particular embodiments the biomaterial may be a surgical implant, prosthesis, scaffold, wound or burn dressing, or the like suitably impregnated, coated or otherwise comprising an isolated protein complex of the invention.

### BRIEF DESCRIPTION OF THE FIGURES

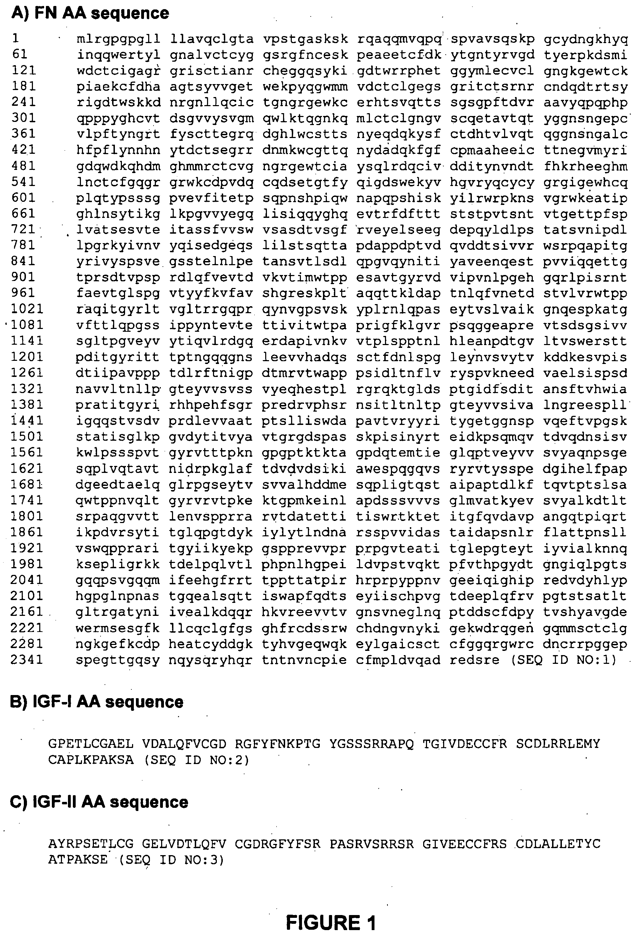

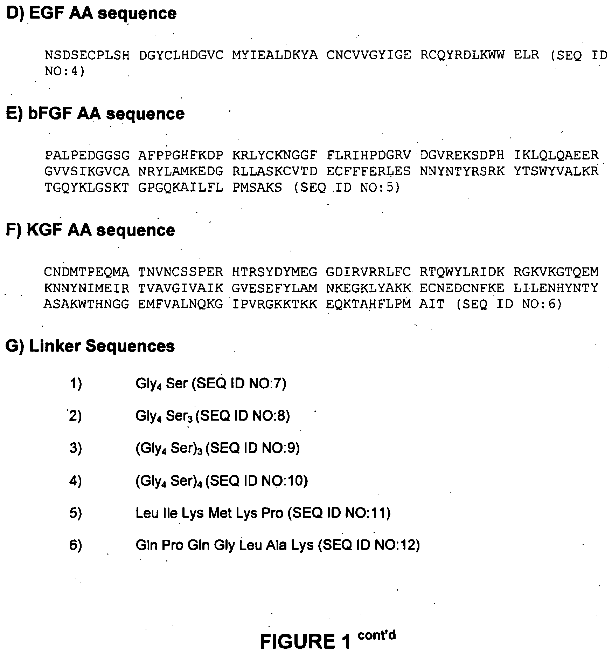

Figure 1. Amino acid sequence of (A) human fibronectin (SEQ ID NO:1), (B) mature IGF-I (SEQ ID NO:2), (C) mature IGF-II (SEQ ID NO:3), (D) mature EGF (SEQ ID NO:4), (E) mature bFGF (SEQ ID NO:5), (F) mature KGF (SEQ ID NO:6), and (G) preferred linker sequences (SEQ ID NOs:7-12).

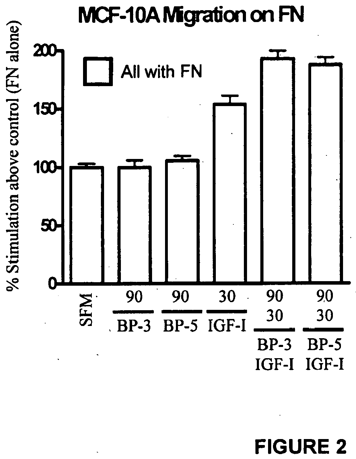

Figure 2. IGF-I, IGFBP and FN protein complexes stimulate breast cancer cell migration. MCF-10A cells were seeded onto Transwells that had been coated with FN (1 µg/mL) and increasing concentrations of IGF-I prebound in the presence of IGFBP-3 or -5. The cells where allowed to migrate for 5 hours. The number of cells traversing the membrane in response to each treatment was then expressed as a percentage of those that migrated on FN only (SFM). MCF-10 data are pooled from three experiments with treatments tested in four wells in each replicate experiment. Error bars indicate SEM. SFM = Serum-free media.

### DETAILED DESCRIPTION OF THE INVENTION

The present invention has arisen from the discovery that synthetic chimeras comprising growth factors such as IGF-I, IGF-II, EGF, bFGF, or KGF and FN bind and exert their biological effect on cell migration through their cognate growth factor receptors and the FN-binding integrin receptor expressed by responsive cells. More particularly, this dual binding event synergistically stimulates cell migration and/or proliferation. These stable, biologically active single-chain chimeric molecules comprise at least the minimal domain or region of a growth factor capable of binding its cognate receptor in combination with one or more type-III domains of FN comprising at least an integrin-binding domain of FN.

This discovery has led the present inventors to provide an isolated protein complex that comprises at least the minimal domain or region of IGF-I, IGF-II, EGF, bFGF, or KGF, for example, capable of binding their cognate receptors, in combination with the integrin-binding domain of FN. Even more particularly, a single, contiguous protein may be produced which comprises these domains.

Such protein complexes, in the form of a single synthetic protein, coordinately bind or co-ligate their cognate receptors and the FN-binding integrin receptor and are therefore useful agents for the promotion of cell migration and/or proliferation and wound healing. Analogously, prevention of cognate receptor and FN-binding integrin receptor co-ligation can be used to prevent cancer cell metastasis.

Throughout this specification, unless otherwise indicated, *"comprise", "comprises"* and "*comprising*" are used inclusively rather than exclusively, so that a stated integer or group of integers may include one or more other non-stated integers or groups of integers.

In the particular context of growth factor receptor-binding domains and integrin-binding domains, such a domain will comprise an amino acid sequence of the domain, together with other, additional amino acids as desired.

It will be understood also that such a domain may *"consist essentially"* of the amino acid sequence of the domain, together with no more than ten, preferably no more than five or even more preferably no more than four, three, two, or one additional amino acids.

It will be understood also that such a domain may *"consist of"* the amino acid sequence of the domain, in the absence of any additional amino acids.

For the purposes of this invention, by *"isolated"* is meant material that has been removed from its natural state or otherwise been subjected to human manipulation. Isolated material may be substantially or essentially free from components that normally accompany it in its natural state, or may be manipulated so as to be in an artificial state together with components that normally accompany it in its natural state. Isolated material may be in native, chemical synthetic or recombinant form.

As used herein, by *"synthetic"* is meant not naturally occurring but made through human technical intervention. In the context of synthetic proteins and nucleic acids, this encompasses molecules produced by recombinant, chemical synthetic or combinatorial techniques as are well understood in the art.

By *"protein"* is meant an amino acid polymer. The amino acids may be natural or non-natural amino acids, D- or L- amino acids as are well understood in the art. The term *"protein"* also includes and encompasses such terms as *"glycoprotein", "lipoprotein"* and the like, as are commonly used in the art.

A *"peptide"* is a protein having less than fifty (50) amino acids.

A "*polypeptide*" is a protein having fifty (50) or more amino acids.

As hereinbefore described, the present invention provides, in one particular aspect, an isolated protein complex in the form of a synthetic chimeric protein comprising an amino acid sequence of:
(i) a growth factor, or at least a domain of a growth factor which is capable of binding a cognate growth factor receptor; and
(ii) one or more type-III domains of fibronectin (FN), wherein the one or more type-III domains of FN consist of amino acids 1266-1536 of the FN sequence set forth in SEQ ID NO: 1, or amino acids 1447-1536 of the FN sequence set forth in SEQ ID NO:1, or amino acids 1173-1536 of the FN sequence set forth in SEQ ID NO:1, which one or more type-III domains of FN comprise an integrin-binding domain.

As used herein, a *"chimeric protein",* comprises a contiguous sequence of amino acids, the amino acids derived from an integrin receptor-binding domain of fibronectin, optionally, additional domains of fibronectin, and a growth factor or at least a receptor-binding domain of a growth factor.

As used herein, a *"growth factor"* is a biologically active protein that is capable of regulating cell growth, differentiation, survival and/or migration *in vitro* and/or *in vivo.*

Exemplary growth factors include, but are not limited to, IGFs (Jones & Clemmons, 1995, Endocrine Rev. 16 3; Wood & Yee, 2000, J. Mammary Gland Biology and Neoplasia 5 1; Keiss et al., 1994, Hormone Research 41 66), such as IGF-I (UniProtKB/Swiss-Prot: #P05019, mature protein comprises amino acid residues 49-118 of the complete sequence) and IGF-II (UniProtKB/Swiss-Prot: #P01344, mature protein comprises amino acid residues 25-91 of the complete sequence), VEGF (Neufeld et al., 1999, FASEB J. 13 9-22), PDGF (Heldin, 1992, EMBO J. 11 4251-4259), EGF (Heldin et al., 1981, Science 4 1122-1123; UniProtKB/Swiss-Prot: #P01133, mature protein comprises amino acid residues 971-1023 of the complete sequence), fibroblast growth factor (FGF; Nurcombe et al., 2000, J. Biol. Chem. 275 30009-30018), bFGF (Taraboletti et al., 1997, Cell Growth. Differ. 8 471-479; UniProtKB/Swiss-Prot: #P09038, mature protein comprises amino acid residues 143-288 of the complete sequence), osteopontin (Nam et al., 2000, Endocrinol. 141 1100), thrombospondin-1 (Nam *et al.,* 2000, *supra*), tenascin-C (Arai et al, 1996, J. Biol. Chem. 271 6099), PAI-1 (Nam et al., 1997, Endocrinol. 138 2972), plasminogen (Campbell et al., 1998, Am. J. Physiol. 275 E321), fibrinogen (Campbell et al., 1999, J. Biol. Chem 274 30215**),** fibrin (Campbell *et al.,* 1999, *supra),* transferrin (Weinzimer et al., 2001, J. Clin. Endocrinol. Metab. 86 1806), and KGF (Marchese et al., 1990, J. Cell Physiol. 144 326-32; UniProtKB/Swiss-Prot: #P21781, mature protein comprises amino acid residues 32-194 of the complete sequence).

Isolated protein complexes in the form of synthetic chimeric proteins of the invention comprise a growth factor or at least a domain of a growth factor which is capable of binding a cognate growth factor receptor.

In this context, by *"domain"* is meant at least that portion or region of a growth factor that is capable of binding a cognate growth factor receptor. Typically, although not exclusively, the cognate growth factor receptor is expressed by a cell and binding or ligation of said cognate growth factor receptor by said at least a domain of a growth factor elicits a cellular response such as cell growth, differentiation, survival and/or migration.

With particular regard to IGF-I, said domain suitably comprises amino acid residue 24, which is not a leucine residue.

Typically, said residue is tyrosine.

With particular regard to IGF-II, said domain suitably comprises amino acid residue 27, which is not a leucine residue.

Typically, said residue is tyrosine.

With particular regard to IGF-I, in one embodiment said domain consists of residues 1 to 70 of IGF-I.

In another embodiment, said domain consists of residues 4 to 70 of IGF-I.

It will also be understood that another component of the isolated protein complexes of the invention isone or more type-III domains of fibronectin (FN), wherein the one or more type-III domains of FN consist of amino acids 1266-1536 of the FN sequence set forth in SEQ ID NO: 1, or amino acids 1447-1536 of the FN sequence set forth in SEQ ID NO:1, or amino acids 1173-1536 of the FN sequence set forth in SEQ ID NO:1, which one or more type-III domains of FN comprise an integrin-binding domain.

Preferably, the integrin-binding domain is an α₁ integrin-binding domain or an α₄ integrin-binding domain.

Although not wishing to be bound by any particular theory, it is proposed that synthetic chimeric proteins are able to co-ligate and co-activate a cognate receptor for said growth factor and an integrin receptor for fibronectin to thereby stimulate, induce, augment, or otherwise promote cell migration.

An advantage of chimeric proteins according to the invention is that they are readily produced by chemical synthetic or recombinant means and are expected to be more stable *in vivo,* as they do not rely on maintaining the protein-protein interactions that are required in non-covalently associated oligo-protein complexes.

In this regard, although isolated protein complexes that comprise receptor-binding domains of IGF-I would also comprise an IGFBP, it is proposed that according to the aforementioned mode of action, an IGFBP is preferably not present in an IGF-I/FN synthetic chimera.

In other embodiments, the invention provides isolated protein complexes, such as in the form of synthetic chimeric proteins, comprising IGF-I, IGF-II, EGF, bFGF, or KGF and one or more type-III domains of FN, wherein the one or more type-III domains of FN consist of amino acids 1266-1536 of the FN sequence set forth in SEQ ID NO:1, or amino acids 1447-1536 of the FN sequence set forth in SEQ ID NO:1, or amino acids 1173-1536 of the FN sequence set forth in SEQ ID NO:1, which one or more type-III domains of FN comprise an integrin-binding domain.

Preferably, the integrin-binding domain is an α₁ integrin-binding domain or an α₄ integrin-binding domain.

The integrin-binding domain of fibronectin suitably comprises an RGD sequence. The RGD sequence is located in fibronectin type III domains 8 to 10 (amino acids 1266-1536 of the fibronectin sequence). More specifically, the RGD sequence is present in the fibronectin type III domain 10, defined by amino acids 1447-1536 of the fibronectin sequence, although secondary integrin-binding sites may be present across the larger 8 to 10 domain region.

Accordingly, in one particular embodiment, the synthetic chimera comprises a fibronectin fragment comprising an RGD sequence, wherein the fragment comprises or consists of amino acids 1447-1536 of a fibronectin amino acid sequence.

In another particular embodiment, the synthetic chimera comprises a fibronectin fragment comprising an RGD sequence, said fragment comprising or consisting of an amino acid sequence of amino acids 1266-1536 of a fibronectin amino acid sequence.

In yet another particular embodiment, the synthetic chimera comprises a fibronectin fragment comprising an RGD sequence according to the aforementioned embodiments, wherein said synthetic chimera further comprises at least 10, 20, 50, 100, 200, 300, 500, 800, or 1000 amino acids of a fibronectin amino acid sequence, for example N-terminal of residue 1266 and/or C-terminal of residue 1536. Thus, said synthetic chimera can include fibronectin type I and/or type II domains, such as, for example, a fibronectin fragment comprising or consisting of at least 6, at least 10, at least 20, at least 50, at least 100, at least 150, at least 200, or all of amino acids 50-273 of a fibronectin amino acid sequence.

In this context, by *"fragment"* is meant a domain, sub-sequence or portion of fibronectin. The fragment preferably constitutes less than 500, less than 400, less than 300 or more preferably about 80-280 contiguous amino acids of a mature fibronectin sequence. Multiple fragments of fibronectin are also contemplated.

In still another particular embodiment, the synthetic chimera comprises a fibronectin fragment comprising or consisting of an amino acid sequence of amino acids 1173 to 1536 of a fibronectin amino acid sequence.

It will be appreciated that the foregoing fibronectin sequence numbering is made with reference to the fibronectin sequence shown in FIG. 1. This fibronectin sequence is derived from the UniProtKB Protein Database, protein accession number P02751. Fibronectin domains and regions are set forth in Table I.

Preferably, synthetic chimeras comprising fibronectin or a fragment comprising an integrin-binding domain do not comprise an IGFBP amino acid sequence.

Preferably, synthetic chimeric proteins as hereinbefore described further comprise a *"linker sequence"* located between and contiguous with a growth factor sequence and a fibronectin amino acid sequence.

In one embodiment, said linker sequence comprises one or more glycine residues and one or more serine residues.

Particular examples of linker sequences may be selected from; Gly₄ Ser (SEQ ID NO:7); Gly₄ Ser₃ (SEQ ID NO:8); (Gly₄ Ser)₃ (SEQ ID NO:9); and (Gly₄ Ser)₄ (SEQ ID NO: 10), although without limitation thereto.

In another embodiment, the linker sequence includes a Plasmin Cleavage Recognition Site (Sakiyama-Elbert et al., 2001, FASEB 15 1300), such as according to the sequence:
Leu Ile Lys Met Lys Pro (SEQ ID NO: 11)

In yet another embodiment, the linker sequence includes a Collagenase-3 Cleavage Recognition Site (Kim & Healy, 2003, Biomacromolecules 4 1214), such as according to the sequence:
Gln Pro Gln Gly Leu Ala Lys (SEQ ID NO: 12)

Biologically-active fragments of the synthetic chimeric proteins of the invention and/or biologically-active fragments of the particular growth factor receptor-binding domains and integrin- binding domains exemplified herein may be useful.

Said *"biologically-active fragment"* may have no less than 10%, preferably no less than 25%, more preferably no less than 50% and even more preferably no less than 75%, 80%, 85%, 90%, or 95% of a biological activity of a protein from which it is derived.

Said *"biologically-active fragment"* may alternatively have no less than 10%, preferably no less than 25%, more preferably no less than 50% and even more preferably no less than 75%, 80%, 85%, 90%, or 95% of a contiguous amino acid sequence of a protein from which it is derived.

Also contemplated are variant protein complexes of the invention.

Typically, and in relation to proteins, a *"variant"* protein has one or more amino acids that have been replaced by different amino acids. It is well understood in the art that some amino acids may be changed to others with broadly similar properties without changing the nature of the activity of the protein (conservative substitutions).

It will be appreciated that one or more amino acid residues of a reference sequence, such as a growth factor, receptor-binding domain of a growth factor, an integrin-binding domain of fibronectin, IGFBPs, or one or more corresponding residues present in a synthetic chimeric protein, may be modified or deleted, or additional sequences added, without substantially altering the biological activity of the isolated protein complex of the invention.

In one embodiment, a protein variant shares at least 70%, preferably at least 80% or 85% and more preferably at least 90%, 95%, 98%, or 99% sequence identity with a reference amino acid sequence.

Preferably, sequence identity is measured over at least 60%, more preferably over at least 75%, more preferably over at least 90% or more preferably over at least 95%, 98% or substantially the full length of the reference sequence.

In order to determine percent sequence identity, optimal alignment of amino acid and/or nucleotide sequences may be conducted by computerised implementations of algorithms (Geneworks program by Intelligenetics; GAP, BESTFIT, FASTA, and TFASTA in the Wisconsin Genetics Software Package Release 7.0, Genetics Computer Group, WI, USA) or by inspection and the best alignment (*i.e.,* resulting in the highest percentage homology over the comparison window) generated by any of the various methods selected. Reference also may be made to the BLAST family of programs as for example disclosed by Altschul et al., 1997, Nucl. Acids Res. 25 3389.

In another example, *"sequence identity"* may be understood to mean the "match percentage" calculated by the DNASIS computer program (Version 2.5 for windows; available from Hitachi Software engineering Co., Ltd., South San Francisco, California, USA).

A detailed discussion of sequence analysis can be found in Unit 19.3 of CURRENT PROTOCOLS IN MOLECULAR BIOLOGY Eds. Ausubel et al. (John Wiley & Sons Inc NY, 1995- 1999).

The invention also contemplates derivatives of a receptor-binding domain of a growth factor, an integrin-binding domain of fibronectin or an isolated protein complex comprising the same.

As used herein, *"derivative"* proteins of the invention have been altered, for example by addition, conjugation or complexing with other chemical moieties or by post-translational modification techniques as are well understood in the art.

*"Additions"* of amino acids may include fusion of the polypeptides or variants thereof with other polypeptides or proteins. The other protein may, by way of example, assist in the purification of the protein. For instance, these include a polyhistidine tag, maltose binding protein, green fluorescent protein (GFP), Protein A, or glutathione S-transferase (GST).

Other derivatives contemplated by the invention include, but are not limited to, modification to side chains, incorporation of unnatural amino acids and/or their derivatives during peptide, polypeptide or protein synthesis and the use of crosslinkers and other methods which impose conformational constraints on the polypeptides, fragments and variants of the invention. Examples of side chain modifications contemplated by the present invention include modifications of amino groups such as by acylation with acetic anhydride, acylation of amino groups with succinic anhydride and tetrahydrophthalic anhydride, amidination with methylacetimidate, carbamoylation of amino groups with cyanate, pyridoxylation of lysine with pyridoxal-5-phosphate followed by reduction with NaBK₄, reductive alkylation by reaction with an aldehyde followed by reduction with NaBK₄, and trinitrobenzylation of amino groups with 2, 4, 6-trinitrobenzene sulphonic acid (TNBS).

The carboxyl group may be modified by carbodiimide activation via O-acylisourea formation followed by subsequent derivitization, by way of example, to a corresponding amide.

The guanidine group of arginine residues may be modified by formation of heterocyclic condensation products with reagents such as 2,3-butanedione, phenylglyoxal and glyoxal.

Sulphydryl groups may be modified by methods such as performic acid oxidation to cysteic acid, formation of mercurial derivatives using 4-chloromercuripheriylsulphonic acid, 4-chloromercuribenzoate, 2-chloromercuri-4-nitrophenol, phenylmercury chloride, and other mercurials, formation of a mixed disulphides with other thiol compounds, reaction with maleimide, maleic anhydride or other substituted maleimide, carboxymethylation with iodoacetic acid or iodoacetamide, and carbamoylation with cyanate at alkaline pH.

Tryptophan residues may be modified, for example, by alkylation of the indole ring with 2-hydroxy-5-nitrobenzyl bromide or sulphonyl halides, or by oxidation with N-bromosuccinimide.

Tyrosine residues may be modified by nitration with tetranitromethane to form a 3-nitrotyrosine derivative.

The imidazole ring of a histidine residue may be modified by N-carbethoxylation with diethylpyrocarbonate or by alkylation with iodoacetic acid derivatives.

Examples of incorporating non-natural amino acids and derivatives during peptide synthesis include, but are not limited to, use of 4-amino butyric acid, 6-aminohexanoic acid, 4-amino-3-hydroxy-5-phenylpentanoic acid, 4-amino-3-hydroxy-6-methylheptanoic acid, t-butylglycine, norleucine, norvaline, phenylglycine, ornithine, sarcosine, 2-thienyl alanine, and/or D-isomers of amino acids.

An example of methods suitable for chemical derivatization of proteins is provided in Chapter 15 of CURRENT PROTOCOLS IN PROTEIN SCIENCE Eds. Coligan et al., John Wiley & Sons NY (1995-2001).

Isolated protein complexes, and individual protein components thereof, (inclusive of fragments, variants, derivatives, and homologs) may be prepared by any suitable procedure known to those of skill in the art.

Proteins of the invention may be produced by chemical synthesis. Chemical synthesis techniques are well known in the art, although the skilled person may refer to Chapter 18 of CURRENT PROTOCOLS IN PROTEIN SCIENCE Eds. Coligan et al., John Wiley & Sons NY (1995-2001) for examples of suitable methodology.

Proteins may alternatively be prepared as recombinant proteins.

While production of recombinant proteins is well known in the art, the skilled person may refer to standard protocols as for example described in Sambrook et al., MOLECULAR CLONING. A Laboratory Manual (Cold Spring Harbor Press, 1989), in particular Sections 16 and 17; CURRENT PROTOCOLS IN MOLECULAR BIOLOGY Eds. Ausubel et al., (John Wiley & Sons, Inc. 1995-1999), in particular Chapters 10 and 16; and CURRENT PROTOCOLS IN PROTEIN SCIENCE Eds. Coligan et al., (John Wiley & Sons, Inc. 1995-1999), in particular Chapters 1, 5 and 6.

The recombinant protein may be produced by a method including the steps of:
(i) preparing an expression construct which comprises a nucleic acid encoding said protein, operably linked to one or more regulatory nucleotide sequences in an expression vector;
(ii) transfecting or transforming a host cell with the expression construct; and
(iii) expressing the recombinant protein in said host cell.

An *"expression vector"* may be either a self-replicating extra-chromosomal vector such as a plasmid, or a vector that integrates into a host genome.

By *"operably linked"* or *"operably connected"* is meant that said regulatory nucleotide sequence(s) is/are positioned relative to the recombinant nucleic acid of the invention to initiate, regulate or otherwise control transcription of the nucleic acid, or translation of a protein encoded by the nucleic acid.

Regulatory nucleotide sequences will generally be appropriate for the host cell used for expression. Numerous types of appropriate expression vectors and suitable regulatory sequences are known in the art for a variety of host cells.

Typically, said one or more regulatory nucleotide sequences may include, but are not limited to, promoter sequences, leader or signal sequences, ribosomal binding sites, transcriptional start and termination sequences, translational start and termination sequences, splice donor/acceptor sequences, and enhancer or activator sequences.

Constitutive promoters (such as CMV, RSV, adenovirus, SV40, and human elongation factor promoters) and inducible/repressible promoters (such as *tet-*repressible promoters and IPTG-, metallothionine- or ecdysone-inducible promoters) are well known in the art and are contemplated by the invention. It will also be appreciated that promoters may be hybrid promoters that combine elements of more than one promoter.

The expression construct may also include a fusion partner (typically provided by the expression vector) so that the recombinant protein of the invention is expressed as a fusion polypeptide with said fusion partner. The main advantage of fusion partners is that they assist identification and/or purification of said fusion protein.

Well known examples of fusion partners include, but are not limited to, glutathione-S-transferase (GST), Fc portion of human IgG, maltose binding protein (MBP), and hexahistidine (HIS₆), which are particularly useful for isolation of the fusion protein by affinity chromatography. For the purposes of fusion protein purification by affinity chromatography, relevant matrices for affinity chromatography are glutathione-, amylose-, and nickel- or cobalt-conjugated resins respectively. Many such matrices are available in "kit" form, such as the QIAexpress™ system (Qiagen) useful with (HIS₆) fusion partners and the Pharmacia GST purification system.

In some cases, the fusion partners also have protease cleavage sites, such as for Factor Xₐ or Thrombin, which allow the relevant protease to partially digest the fusion protein of the invention and thereby liberate the recombinant polypeptide of the invention therefrom. The liberated protein can then be isolated from the fusion partner by subsequent chromatographic separation.

Fusion partners also include within their scope *"epitope tags",* which are usually short peptide sequences for which a specific antibody is available. Well known examples of epitope tags for which specific monoclonal antibodies are readily available include c-myc, haemagglutinin and FLAG tags.

Suitable host cells for expression may be prokaryotic or eukaryotic, such as *Escherichia coli* (DH5α for example), yeast cells, Sf9 cells utilized with a baculovirus expression system, CHO cells, COS, CV-1, NIH 3T3, and 293 cells, although without limitation thereto.

Expression constructs may also include one or more selection marker nucleotide sequences that confer transformed host cell resistance to a selection agent. Selection markers useful for the purposes of selection of transformed bacteria include *bla, kanR* and *tetR,* while transformed eukaryotic cells may be selected by markers such as hygromycin, G418 and puromycin, although without limitation thereto.

With regard to introducing genetic material into host cells, the terms *"transforming"* and *"transfecting"* are used generally to describe introduction of genetic material into a host cell. There are many well known methods for introducing foreign genetic material into a host cell including, but not limited to, calcium phosphate precipitation, electroporation, delivery by lipofectamine, lipofectin and other lipophilic agents, calcium phosphate precipitation, DEAE-Dextran transfection, microparticle bombardment, microinjection, and protoplast fusion.

The invention provides an isolated nucleic acid that encodes a synthetic chimeric protein of the invention, including variants and homologs thereof.

The term *"nucleic acid"* as used herein designates single-or double-stranded mRNA, RNA, cRNA, RNAi, and DNA, inclusive of cDNA and genomic DNA and DNA-RNA hybrids.

A "*polynucleotide*" is a nucleic acid having eighty (80) or more contiguous nucleotides, while an *"oligonucleotide"* has less than eighty (80) contiguous nucleotides.

A *"probe"* may be a single or double-stranded oligonucleotide or polynucleotide, suitably labeled for the purpose of detecting complementary sequences in Northern or Southern blotting, for example.

A *"primer"* is usually a single-stranded oligonucleotide, preferably having 15-50 contiguous nucleotides, which is capable of annealing to a complementary nucleic acid "template" and being extended in a template-dependent fashion by the action of a DNA polymerase such as *Taq* polymerase, RNA-dependent DNA polymerase or Sequenase™.

Synthetic nucleic acids of the invention may be produced by chemical synthetic approaches or by recombinant methods that utilize nucleic acid sequence amplification techniques, or a combination thereof, as are well known in the art.

Chemically synthesized primers and oligonucleotides, synthesizers and associated technologies useful according to the present invention are typically available in most laboratories or may be purchased from commercial sources.

Suitable nucleic acid amplification techniques are well known to the skilled person, and include polymerase chain reaction (PCR) and ligase chain reaction (LCR) as for example described in Chapter 15 of Ausubel *et al. supra;* strand displacement amplification (SDA) as for example described in U.S. Patent No 5,422,252; rolling circle replication (RCR) as for example described in Liu et al., 1996, J. Am. Chem. Soc. 118 1587, International application WO 92/01813 and International Application WO 97/19193; nucleic acid sequence-based amplification (NASBA) as for example described by Sooknanan et al., 1994, Biotechniques 17 1077; and Q-β replicase amplification as for example described by Tyagi et al., 1996, Proc. Natl. Acad. Sci. USA 93 5395, although without limitation thereto.

A preferred nucleic acid sequence amplification technique is PCR. As used herein, an *"amplification product"* refers to a nucleic acid product generated by a nucleic acid amplification technique.

In producing and expressing nucleic acids of the invention, it will also be appreciated that advantage may be taken with respect to codon sequence redundancy, such that the nucleic acids exemplified herein may be readily modified without changing an amino acid sequence encoded thereby.

In particular embodiments, nucleic acids may be optimized according to preferred *"codon usage"* of a host cell to be used for recombinant expression, as is well known in the art. This can effectively "tailor" a nucleic acid for optimal expression in a particular organism, or cells thereof, where preferential codon usage affects protein expression.

Therefore, the invention includes synthetic nucleic acids that are homologous to the nucleic acids exemplified herein.

In one embodiment, nucleic acid homologs share at least 70%, preferably at least 80%, more preferably at least 90%, and even more preferably at least 95% sequence identity with a nucleic acid encoding any one of the synthetic chimeric protein constructs described herein.

Preferably, sequence identity is measured over at least 70%, more preferably at least 80%, even more preferably at least 90%, 95% or advantageously over substantially the full length of the encoding nucleic acid of the invention.

In another embodiment, nucleic acid homologs hybridize to a nucleic acid encoding any one of the synthetic chimeric protein constructs described herein under high stringency conditions.

*"Hybridize"* and *"hybridization"* are used herein to denote the pairing of at least partly complementary nucleotide sequences to produce a DNA-DNA, RNA-RNA or DNA-RNA duplex. Hybridized sequences occur through base-pairing between complementary purines and pyrimidines as is well known in the art.

In this regard, it will be appreciated that modified purines (for example, inosine, methylinosine and methyladenosine) and modified pyrimidines (thiouridine and methylcytosine) may also engage in base pairing.

*"Stringency"* as used herein, refers to temperature and ionic strength conditions, and presence or absence of certain organic solvents and/or detergents during hybridisation. The higher the stringency, the higher will be the required level of complementarity between hybridizing nucleotide sequences.

*"Stringent conditions"* designates those conditions under which only nucleic acid having a high frequency of complementary bases will hybridize.

Reference herein to high stringency conditions includes and encompasses:
(i) from at least about 31% v/v to at least about 50% v/v formamide and from at least about 0.01 M to at least about 0.15 M salt for hybridisation at 42°C, and at least about 0.01 M to at least about 0.15 M salt for washing at 42°C;
(ii) 1% BSA, 1 mM EDTA, 0.5 M NaHPO₄ (pH 7.2), 7% SDS for hybridization at 65°C, and (a) 0.1 x SSC, 0.1% SDS; or (b) 0.5% BSA, 1mM EDTA, 40 mM NaHPO₄ (pH 7.2), 1% SDS for washing at a temperature in excess of 65°C for about one hour; and
(iii) 0.2 x SSC, 0.1% SDS for washing at or above 68°C for about 20 minutes.

In general, washing is carried out at Tₘ = 69.3 + 0.41 (G + C) % -12°C. In general, the Tₘ of a duplex DNA decreases by about 1°C with every increase of 1% in the number of mismatched bases.

Notwithstanding the above, stringent conditions are well known in the art, such as described in Chapters 2.9 and 2.10 of. Ausubel *et al., supra* and in particular at pages 2.9.1 through 2.9.20.

Also contemplated are antibodies against a synthetic chimeric protein of the invention, inclusive of chimeric proteins, or fragments, variants and/or derivatives thereof. Antibodies of the invention may be polyclonal or monoclonal. Well-known protocols applicable to antibody production, purification and use may be found, for example, in Chapter 2 of Coligan et al., CURRENT PROTOCOLS IN IMMUNOLOGY (John Wiley & Sons NY, 1991-1994) and Harlow, E. & Lane, D. Antibodies: A Laboratory Manual, Cold Spring Harbor, Cold Spring Harbor Laboratory, 1988.

Generally, said antibodies bind to or conjugate with a polypeptide, fragment, variant or derivative of the invention. For example, the antibodies may comprise polyclonal antibodies. Such antibodies may be prepared for example by injecting a polypeptide, fragment, variant or derivative of the invention into a production species, which may include mice or rabbits, to obtain polyclonal antisera. Methods of producing polyclonal antibodies are well known to those skilled in the art. Exemplary protocols which may be used are described for example in Coligan et al., CURRENT PROTOCOLS IN IMMUNOLOGY, *supra,* and in Harlow & Lane, 1988, *supra.*

In lieu of the polyclonal antisera obtained in the production species, monoclonal antibodies may be produced using the standard method as for example, described by Köhler & Milstein (1975, Nature 256, 495), or by more recent modifications thereof as, for example, described in Coligan et al., CURRENT PROTOCOLS IN IMMUNOLOGY, *supra* by immortalizing spleen or other antibody producing cells derived from a production species which has been inoculated with one or more of the polypeptides, fragments, variants or derivatives of the invention.

Said antibodies may comprise Fc or Fab fragments of the polyclonal or monoclonal antibodies referred to above. Alternatively, the antibodies may comprise single chain Fv antibodies (scFvs) against the proteins of the invention. Such scFvs may be prepared, for example, in accordance with the methods described respectively in U.S. Patent 5,091,513, European Patent 239,400 or the article by Winter & Milstein (1991, Nature 349 293).

Labels may be associated with the antibody or antibody fragment.

The label may be selected from a group including a chromogen, a catalyst, an enzyme, a fluorophore, a chemiluminescent molecule, a lanthanide ion such as Europium (Eu³⁴), a radioisotope, and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes useful as labels are disclosed in U.S. Patent 4,366,241, U.S. Patent 4,843,000 and U.S. Patent 4,849,338. Enzyme labels useful in the present invention include alkaline phosphatase, horseradish peroxidase, luciferase, b-galactosidase, glucose oxidase, lysozyme, malate dehydrogenase, and the like. The enzyme label may be used alone or in combination with a second enzyme in solution.

By way of example, the fluorophore may be fluorescein isothiocyanate (FITC), Oregon green, tetramethylrhodamine isothiocyanate (TRITL), allophycocyanin (APC), and R-Phycoerythrin (RPE), although without limitation thereto.

The invention also provides pharmaceutical compositions that comprise an isolated protein complex of the invention, inclusive of variants and derivatives thereof.

Such isolated protein complex may be in any form, inclusive of synthetic chimeric proteins of the invention, although without limitation thereto.

Pharmaceutical compositions of the invention may be used to promote or otherwise facilitate cell migration, tissue regeneration and wound healing. Alternatively, pharmaceutical compositions may be administered to prevent tumour metastasis by preventing or inhibiting tumour cell migration to a secondary site.

The composition may be used in therapeutic or prophylactic treatments as required. For example, pharmaceutical compositions may be applied in the form of therapeutic or cosmetic preparations for skin repair, wound healing, healing of burns and other dermatological treatments.

In this regard, pharmaceutical compositions may be administered in association with, or as a component of, a biomaterial, biopolymer, inorganic material such as hydroxyapatite or derivatives thereof, surgical implant, prosthesis, wound or burn dressing, compress, bandage, or the like suitably impregnated, coated or otherwise comprising the pharmaceutical composition.

Suitably, the pharmaceutical composition comprises an appropriate pharmaceutically-acceptable carrier, diluent or excipient.

Preferably, the pharmaceutically-acceptable carrier, diluent or excipient is suitable for administration to mammals, and more preferably, to humans.

By *"pharmaceutically-acceptable carrier, diluent or excipient"* is meant a solid or liquid filler, diluent or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates, and pyrogen-free water.

A useful reference describing pharmaceutically acceptable carriers, diluents and excipients is Remington's Pharmaceutical Sciences (Mack Publishing Co. N.J. USA, 1991).

Any safe route of administration may be employed for providing a patient with the composition of the invention. For example, oral, rectal, parenteral, sublingual, buccal, intravenous, intra-articular, intra-muscular, intra-dermal, subcutaneous, inhalational, intraocular, intraperitoneal, intracerebroventricular, transdermal, and the like may be employed.

Dosage forms include tablets, dispersions, suspensions, injections, solutions, syrups, troches, capsules, suppositories, aerosols, transdermal patches, and the like. These dosage forms may also include injecting or implanting controlled releasing devices designed specifically for this purpose or other forms of implants modified to act additionally in this fashion. Controlled release of the therapeutic agent may be effected by coating the same, for example, with hydrophobic polymers including acrylic resins, waxes, higher aliphatic alcohols, polylactic and polyglycolic acids, and certain cellulose derivatives such as hydroxypropylmethyl cellulose. In addition, the controlled release may be effected by using other polymer matrices, liposomes and/or microspheres.

The above compositions may be administered in a manner compatible with the dosage formulation, and in such amount as is pharmaceutically-effective. The dose administered to a patient, in the context of the present invention, should be sufficient to effect a beneficial response in a patient over an appropriate period of time. The quantity of agent(s) to be administered may depend on the subject to be treated, inclusive of the age, sex, weight and general health condition thereof, factors that will depend on the judgement of the practitioner.

With regard to pharmaceutical compositions for wound healing, particular reference is made to U.S. Patent 5,936,064 and International Publication WO 99/62536.

Pharmaceutical compositions of the invention may also include expression vectors such as viral vectors such as vaccinia, and viral vectors useful in gene therapy. The latter include adenovirus and adenovirus-associated viruses (AAV) such as described in Braun-Falco et al. (1999, Gene Ther. 6 432), retroviral and lentivral vectors such as described in Buchshacher et al. (2000, Blood 95 2499) and vectors derived from herpes simplex virus and cytomegalovirus. A general overview of viral vectors useful in endocrine gene therapy is provided in Stone et al. (2000, J. Endocrinol. 164 103).

The present invention may also utilize specific expression vectors which target gene expression to epidermal cells, such as described in U.S. Patent 5,958,764 and for *in vivo* wound healing applications, such as described in U.S. Patent 5,962,427.

The invention provides an isolated protein complex for use in wound or burn healing in an animal. This may particularly include the therapeutic and/or prophylactic treatment of mammals, and more particularly, humans.

However, therapeutic uses according to the invention may also be applicable to mammals such as domestic and companion animals, performance animals such as horses, camels and greyhounds, livestock, laboratory animals and animals used as sources of cells, organs and tissues for xenotransplantation.

Also contemplated are methods of cosmetic treatment where isolated protein complexes, inclusive of synthetic chimeric proteins of the invention, are administered to improve or enhance skin quality or skin appearance.

Such treatments may include prevention or remediation of skin disorders such as psoriasis and hypertrophic scarring that result from aberrant skin cell proliferation.

Alternatively, methods of treatment are contemplated whereby tumour metastasis is prevented or inhibited by blocking tumour cell migration to a secondary site. In addition, methods of treating cancer by blocking cell proliferation are also contemplated.

In particular embodiments, therapeutic and/or prophylactic treatments may utilize an isolated protein complex, inclusive of synthetic chimeric proteins of the invention, in association with, or as a component of, a biomaterial, biopolymer, inorganic material such as fluorohydroxyapatite, surgical implant, prosthesis, wound or burn dressing, compress, bandage, or the like suitably impregnated, coated or otherwise comprising the isolated protein complex.

Such methods include administration of pharmaceutical compositions as hereinbefore defined, and may be by way of microneedle injection into specific tissue sites, such as described in U.S. Patent 6,090,790, topical creams, lotions or sealant dressings applied to wounds, burns or ulcers, such as described in U.S. Patent 6,054,122 or implants which release the composition such as described in International Publication WO 99/47070.

Gene therapy is also applicable in this regard, such as according to methods set forth in U.S. Patent 5,929,040 and U.S. Patent 5,962,427.

There also exist methods by which skin cells can be genetically modified for the purpose of creating skin substitutes, such as by genetically engineering desired growth factor expression (Supp et al., 2000, J. Invest. Dermatol. 114 5). An example of a review of this field is provided in Bevan et al. (Biotechnol. Gent. Eng. Rev. 16 231).

Also contemplated is "seeding" a recipient with transfected or transformed cells, such as described in International Publication WO 99/11789.

These methods can be used to stimulate cell migration and thereby facilitate or progress wound and burn healing, repair of skin lesions such as ulcers, tissue replacement and grafting such as by *in vitro* culturing of autologous skin, re-epithelialization of internal organs such as kidney and lung and repair of damaged nerve tissue.

Skin replacement therapy has become well known in the art, and may employ use of co-cultured epithelial/keratinocyte cell lines, for example as described in Kehe et al. (1999, Arch. Dermatol. Res. 291 600) or *in vitro* culture of primary (usually autologous) epidermal, dermal and/or keratinocyte cells. These techniques may also utilize engineered biomaterials and synthetic polymer "scaffolds".

Examples of reviews of the field in general are provided in Terskilch & Vasiliev (1999, Int. Rev. Cytol. 188 41) and Eaglestein & Falanga (1998, Cutis 62 1).

More particularly, the production of replacement oral mucosa useful in craniofacial surgery is described in Izumi et al. (2000, J. Dent. Res. 79 798). Fetal keratinocytes and dermal fibroblasts can be expanded *in vitro* to produce skin for grafting to treat skin lesions, such as described in Fauza et al. (J. Pediatr. Surg. 33 357), while skin substitutes from dermal and epidermal skin elements cultured *in vitro* on hyaluronic acid-derived biomaterials have been shown to be potentially useful in the treatment of burns (Zacchi et al., 1998, J. Biomed. Mater. Res. 40 187).

Polymer scaffolds are also contemplated for the purpose of facilitating replacement skin engineering, as for example described in Sheridan et al. (2000, J. Control Release 14 91) and Fauza *et al.* (1998, *supra*), as are microspheres as agents for the delivery of skin cells to wounds and burns (LaFrance & Armstrong, 1999, Tissue Eng. 5 153).

Also contemplated is use of isolated protein complexes, inclusive of synthetic chimeric proteins of the invention, to identify, screen, design or otherwise produce agonists or antagonists of complexes comprising a growth factor and fibronectin, such as IGF-I:FN, IGF-II:FN, EGF:FN, bFGF:FN, GF:FN, or IGF-I:IGFBP:FN complexes. Such agents may be a *"mimetic".* The term *"mimetic"* is used herein to refer to molecules that are designed to resemble particular functional regions of proteins or peptides, and includes within its scope the terms *"agonist", "analogue"* and *"antagonist"* as are well understood in the art.

Agonists may be produced that mimic the binding of the cognate growth factor receptors and FN receptors by IGF-I:FN, IGF-II:FN, EGF:FN, bFGF:FN, KGF:FN, or IGF-I:IGFBP:FN complexes. Such molecules may have utility as stimulators of cell migration such as required for wound healing, skin regeneration and the like.

Alternatively, antagonists may be produced that prevent or inhibit the binding of the cognate growth factor receptors and integrin receptors by IGF-I:FN, IGF-II:FN, EGF:FN, bFGF:FN, KGF:FN, or IGFII:IGFBP:FN complexes. Such molecules may have utility as inhibitors of cell migration and/or cell proliferation and thereby constitute useful anti-tumour agents and also in treatments of skin disorders such as psoriasis and hypertrophic scarring that result from aberrant cell proliferation.

The aforementioned mimetics, agonists, antagonists, and analogues may be peptides, polypeptides or other organic molecules, preferably small organic molecules, with a desired biological activity and half-life.

Computer-assisted structural database searching is becoming increasingly utilized as a procedure for identifying mimetics. Database searching methods which, in principle, may be suitable for identifying mimetics, may be found in International Publication WO 94/18232 (directed to producing HIV antigen mimetics), U.S. Patent 5,752,019 and International Publication WO 97/41526 (directed to identifying EPO mimetics).

Other methods include a variety of biophysical techniques which identify molecular interactions. These allow for the screening of candidate molecules according to whether said candidate molecule affects formation of IGF-I:FN, IGF-II:FN, EGF:FN, bFGF:FN, KGF:FN, or IGF-IGFBP-FN complexes, for example. Methods applicable to potentially useful techniques such as competitive radioligand binding assays (see, Upton *et al.,* 1999, *supra* for a relevant method), analytical ultracentrifugation, microcalorimetry, surface plasmon resonance, and optical biosensor-based methods are provided in Chapter 20 of CURRENT PROTOCOLS IN PROTEIN SCIENCE Eds. Coligan et al., (John Wiley & Sons, 1997).

So that the present invention may be more readily understood and put into practical effect, the skilled person is referred to the following non-limiting examples.

### EXAMPLES

### EXAMPLE 1

### IGF-I, IGFBPs and FN stimulate cell migration

MCF-10A cells were seeded onto Transwells that had been coated with FN (1 ug/mL) and increasing concentrations of IGF-I prebound in the presence of IGFBP-3 or -5. The cells where allowed to migrate for 5 hours. The number of cells traversing the membrane in response to each treatment was then expressed as a percentage of those that migrated on FN only (SFM). MCF-10 data are pooled from three experiments with treatments tested in four wells in each replicate experiment and shown in FIG. 2. Error bars indicate SEM. SFM = Serum-free media. IGF-I:FN, IGF-I:IGFBP-3:FN and IGF-I:IGFBP-5:FN were able to stimulate significantly increased migration above that of FN alone control wells (responses of 153.7 +/- 7.3%, 192.5 +/- 6.8% and 187.5 +/- 6.5% of the FN control wells, respectively) (p<0.05). The response of the MCF7-10A cells to IGF-I:IGFBP-3:FN and IGF-I:IGFBP-5:FN treatments was also significantly greater than those obtained with either IGFBP or IGF-I alone with FN (p<0.05). This data indicates that maximal responses occur when the trimeric IGF-I:IGFBP-3/5:FN complexes are present. This suggests that chimeras containing IGF-I linked to FN activate the FN binding integrins and the cognate growth factor receptor.

### EXAMPLE 2

### Synthetic chimeric fibronectin:growth factor proteins

Provided herein are examples of synthetic chimeric proteins of the invention, in the form of FN:growth factor (*e.g.,* IGF-I, IGF-II, EGF, bFGF, and KGF) chimeras.

The synthetic chimeric proteins include any full-length or truncated forms of FN fused with a growth factor, with or without amino acid residue modifications. In addition, FN and the growth factors may be fused with or without the various peptide linkers.

A series of chimeric expression constructs are designed in which various lengths of the FN protein are linked to the full-length mature IGF-I, IGF-II, EGF, bFGF, or KGF proteins, or at least a domain of the IGF-I, IGF-II, EGF, bFGF, or KGF proteins capable of binding a cognate growth factor receptor. In each case, the FN segments are preferably linked to the IGF-I, IGF-II, EGF, bFGF, or KGF sequence via a linker, for example, a Gly₄ Ser (SEQ ID NO:7) linker, a Gly₄ Ser₃ (SEQ ID NO:8) linker, a (Gly₄ Ser)₃ (SEQ ID NO:9) linker, or a (Gly₄ Ser)₄ (SEQ ID NO: 10) linker.

Exemplary synthetic chimeric proteins include, but are not limited to:
A) FN type-III Domain 8 [Linker] Growth Factor (IGF-I, IGF-II, EGF, bFGF, or KGF);
B) FN type-III Domains 8-9 [Linker] Growth Factor (IGF-I, IGF-II, EGF, bFGF, or KGF);
C) FN type-III Domains 8-10 [Linker] Growth Factor (IGF-I, IGF-II, EGF, bFGF, or KGF);
D) FN type-III Domain 9 [Linker] Growth Factor (IGF-I, IGF-II, EGF, bFGF, or KGF);
E) FN type-III Domains 9-10 [Linker] Growth Factor (IGF-I, IGF-II, EGF, bFGF, or KGF);
F) FN type-III Domain 10 [Linker] Growth Factor (IGF-I, IGF-II, EGF, bFGF, or KGF);
G) FN type-I Domains 1-5 [linker] FN type-III Domain 8 [linker] Growth Factor (IGF-I, IGF-II, EGF, bFGF, or KGF);
H) FN type-I Domains 1-5 [linker] FN type-III Domains 8-9 [linker] Growth Factor (IGF-I, IGF-II, EGF, bFGF, or KGF);
I) FN type-I Domains 1-5 [linker] FN type-III Domains 8-10 [linker] Growth Factor (IGF-I, IGF-II, EGF,. bFGF, or KGF);
J) FN type-I Domains 1-5 [linker] FN type-III Domain 9 [linker] Growth Factor (IGF-I, IGF-II, EGF, bFGF, or KGF);
K) FN type-I Domains 1-5 [linker] FN type-III Domains 9-10 [linker] Growth Factor (IGF-I, IGF-II, EGF, bFGF, or KGF);
L) FN type-I Domains 1-5 [linker] FN type-III Domain 10 [linker] Growth Factor (IGF-I, IGF-II, EGF, bFGF, or KGF);
M) FN type-I Domains 4-5 [linker] FN type-III Domain 8 [linker] Growth Factor (IGF-I, IGF-II, EGF, bFGF, or KGF);
N) FN type-I Domains 4-5 [linker] FN type-III Domains 8-9 [linker] Growth Factor (IGF-I, IGF-II, EGF, bFGF, or KGF);
O) FN type-I Domains 4-5 [linker] FN type-III Domains 8-10 [linker] Growth Factor (IGF-I, IGF-II, EGF, bFGF, or KGF);
P) FN type-I Domains 4-5 [linker] FN type-III Domain 9 [linker] Growth Factor (IGF-I, IGF-II, EGF, bFGF, or KGF);
Q) FN type-I Domains 4-5 [linker] FN type-III Domains 9-10 [linker] Growth Factor (IGF-I, IGF-II, EGF, bFGF, or KGF);
R) FN type-I Domains 4-5 [linker] FN type-III Domain 10 [linker] Growth Factor (IGF-I, IGF-II, EGF, bFGF, or KGF).

Human FN, IGF-I, IGF-II, EGF, bFGF, and KGF gene DNA sequences (SEQ ID NOs: 1-6, respectively) can be codon-optimised for expression in *Spodoptera frugiperda.* The coding sequences can then be cloned into an expression vector incorporating a poly-histidine affinity tag to aid in the purification of the chimeras (*e.g.,* the pIB/V5-His expression vector (Invitrogen)). A nucleotide sequence encoding an amino acid linker as discussed above can be inserted via site-directed mutagenesis PCR. The addition of an Asn to the C-terminus of the linker sequence can be used to generate a Asn-Gly motif with Gly being the first amino acid of the growth factor protein. This motif enables hydroxylamine induced cleavage of the growth factor protein from the chimeras.

The resulting constructs will encode various lengths of the FN protein linked by a linker to the full-length mature IGF-I, IGF-II, EGF, bFGF, or KGF proteins, or at least a domain of the IGF-I, IGF-II, EGF, bFGF, or KGF proteins capable of binding a cognate growth factor receptor. The DNA sequence of all constructs can be verified to ensure that the fidelity of the desired DNA sequences are maintained.

Clones in the pIB/V5-His vector can be used to transfect Sf9 insect cells and transiently-expressed secreted protein is detected in the conditioned media, as assessed by immunoblotting. Briefly, the samples are resolved on SDS-PAGE under reducing conditions and the proteins are transferred onto a nitrocellulose membrane using a semi-dry transfer method. The membrane is interrogated with poly-clonal anti-FN antibodies and the target protein species are then visualized using enhanced chemiluminescence.

Purification of the chimeric proteins is based on Ni-NTA Superflow Agarose (QIAGEN, Australia) affinity chromatography performed according to the manufacturer's instructions. The chimeric proteins are monitored throughout the purification process by SDS-PAGE and western blot analysis using a poly-clonal anti-FN antibody (Calbiochem).

Cells, such as MCF-10A cells, MCF-7 cells, and isolated human epithelial cells, keratinocytes and fibroblasts can be used to examine the effects of the synthetic chimeric proteins on cell migration and/or proliferation. For example, cell migration can be assessed using Transwell™ migration assays, while cell proliferation can be determined using cell proliferation assays well known to one of skill in the art.

## Claims

1. An isolated protein complex in the form of a synthetic chimeric protein, comprising an amino acid sequence of:
(i) a growth factor or at least a domain of said growth factor which is capable of binding a cognate growth factor receptor; and
(ii) one or more type-III domains of fibronectin (FN), wherein the one or more type-III domains of FN consist of amino acids 1266-1536 of the FN sequence set forth in SEQ ID NO:1, or amino acids 1447-1536 of the FN sequence set forth in SEQ ID NO:1, or amino acids 1173-1536 of the FN sequence set forth in SEQ ID NO:1, which one or more type-III domains of FN comprise an integrin-binding domain.

2. The isolated protein complex of Claim 1, wherein said growth factor is selected from insulin-like growth factor-I (IGF-I), insulin-like growth factor-II (IGF-II), epidermal growth factor (EGF), basic fibroblast growth factor (bFGF), and keratinocyte growth factor (KGF).

3. The isolated protein complex of Claim 1 or Claim 2, wherein the integrin-binding domain is an α₁ integrin-binding domain or an α₄ integrin-binding domain.

4. The isolated protein complex of any one of Claims 1-3, which does not comprise an IGFBP amino acid sequence.

5. The isolated protein complex of any one of the preceding claims, further comprising an additional fragment of FN..

6. The isolated protein complex of claim 5, wherein the additional fragment of FN comprises amino acids 50-273 of the FN sequence set forth in SEQ ID NO:1 or amino acids 184-273 of the FN sequence set forth in SEQ ID NO:1.

7. The isolated protein complex of any one of the preceding claims, further comprising at least one linker sequence.

8. The isolated protein complex of claim 7, wherein the linker sequence comprises a protease cleavage site, and/or is selected from the group consisting of:
(i) Gly₄ Ser (SEQ ID NO:7);
(ii) Gly₄ Ser₃ (SEQ ID NO:8);
(iii) (Gly₄ Ser)₃ (SEQ ID NO:9);
(iv) (Gly₄ Ser)₄ (SEQ ID NO:10);
(v) Leu Ile Lys Met Lys Pro (SEQ ID NO:11); and
(vi) Gln Pro Gln Gly Leu Ala Lys (SEQ ID NO:12).

9. An isolated nucleic acid encoding the isolated protein complex of any one of the preceding claims.

10. A genetic construct, comprising the isolated nucleic acid of
Claim 9 operably linked to one or more regulatory nucleotide sequences in a vector, wherein preferably the construct is an expression construct, and the isolated nucleic acid is operably linked to a promoter.

11. A host cell comprising the genetic construct of claim 9 or claim 10.

12. A pharmaceutical composition, comprising the isolated protein complex of any one of Claims 1-8 and a pharmaceutically-acceptable carrier, diluent or excipient.

13. A surgical implant, scaffold or prosthesis impregnated, coated or otherwise comprising the isolated protein complex of any one of Claims 1-8, or a wound or burn dressing, comprising the isolated protein complex of any one of Claims 1-8.

14. An *in vitro* method of promoting cell migration and/or proliferation, including the step of using the isolated protein complex of any one of Claims 1-8 to bind both a growth factor receptor and an integrin receptor expressed by a cell to thereby induce, augment or otherwise promote migration and/or proliferation of said cell.

15. The isolated protein complex of any one of Claims 1-8 for use in wound or burn healing in an animal, wherein preferably the animal is a human.

## Patentansprüche

1. Isolierter Proteinkomplex in der Form eines synthetischen chimären Proteins, umfassend eine Aminosäuresequenz von:
(i) einem Wachstumsfaktor oder mindestens einer Domäne des Wachstumsfaktors, der zur Bindung an einen dazugehörigen Wachstumsfaktorrezeptor in der Lage ist; und
(ii) eine oder mehrere Typ !!!-Domänen von Fibronektin (FN), wobei die eine oder die mehreren Typ III-Domänen aus den Aminosäuren 1266-1536 der in der SEQ ID NO: 1 dargelegten FN-Sequenz oder aus den Aminosäuren 1447-1536 der in der SEQ ID NO: 1 dargelegten FN-Sequenz oder aus den Aminosäuren 1173-1536 der in der SEQ ID NO: 1 dargelegten FN-Sequenz bestehen, wobei die eine oder die mehreren Typ III-Domänen eine Integrin-Bindungsdomäne umfassen.

2. Isolierter Proteinkomplex nach Anspruch 1, wobei der Wachstumsfaktor ausgewählt ist aus Insulinähnlicher Wachstumsfaktor-I (IGF-I), Insulinähnlicher Wachstumsfaktor-II (IGF-II), Epidermaler Wachstumsfaktor (EGF), Basischer Fibroblastenwachstumsfaktor (bFGF) und Keratinozyten-Wachstumsfaktor (KGF).

3. Isolierter Proteinkomplex nach Anspruch 1 oder 2, wobei die Integrin-Bindungsdomäne eine α₁ Integrin-Bindungsdomäne oder eine α₄ Integrin-Bindungsdomäne ist

4. Isolierter Proteinkomplex nach einem der Ansprüche 1 bis 3, der keine IGFBP-Aminosäuresequenz umfasst.

5. Isolierter Proteinkomplex nach einem der vorhergehenden Ansprüche, ferner umfassend ein zusätzliches Fragment von FN.

6. Isolierter Proteinkomplex nach Anspruch 5, wobei das zusätzliche Fragment von FN die Aminosäuren 50 - 273 der in SEQ ID NO: 1 dargelegten FN-Sequenz oder die Aminosäuren 184 - 273 der in SEQ ID NO: 1 dargelegten FN-Sequenz umfasst.

7. Isolierter Proteinkomplex nach einem der vorhergehenden Ansprüche, ferner umfassend mindestens eine Linkersequenz.

8. Isolierter Proteinkomplex nach Anspruch 7, wobei die Linkersequenz eine Proteasen-Spaltungsstelle umfasst und/oder aus der Gruppe ausgewählt ist, bestehend aus:
(i) Gly₄ Ser (SEQ ID NO: 7);
(ii) Gly₄ Ser₃ (SEQ ID NO: 8);
(iii) (Gly₄ Ser)₃ (SEQ ID NO: 9);
(iv) (Gly₄ Ser)₄ (SEQ ID NO: 10);
(v) Leu Ile Lys Met Lys Pro (SEQ ID NO: 11); und
(vi) Gln Pro Gln Gly Leu Ala Lys (SEQ ID NO: 12).

9. Isolierte Nukleinsäure, die für den isolierten Proteinkomplex nach einem der vorhergehenden Ansprüche kodiert.

10. Genetisches Konstrukt, das die isolierte Nukleinsäure nach Anspruch 9 umfasst, welches mit einer oder mehreren regulatorischen Nukleotidsequenzen in einem Vektor funktionell verknüpft ist, wobei vorzugsweise das Konstrukt ein Expressionskonstrukt ist und die isolierte Nukleinsäure funktionell mit einem Promotor verknüpft ist.

11. Wirtszelle, umfassend das genetische Konstrukt nach Anspruch 9 oder 10.

12. Pharmazeutische Zusammensetzung, umfassend den isolierten Proteinkomplex nach einem der Ansprüche 1 bis 8 und einen pharmazeutisch akzeptablen Träger, Verdünnungsmittel oder Exzipienten.

13. Chirurgisches Implantat, Gerüst oder Prothese, die mit dem isolierten Proteinkomplex nach einem der Ansprüche 1 bis 8 imprägniert sind, beschichtet sind oder ihn auf andere Weise aufweisen, oder eine Wund- oder Brandwundenauflage, die den isolierten Proteinkomplex nach einem der Ansprüche 1 bis 8 aufweist.

14. *In vitro*-Verfahren zur Förderung der Zellmigration und/oder -proliferation, umfassend den Schritt des Verwendens des isolierten Proteinkomplexes nach einem der Ansprüche 1 bis 8, um sowohl an einen Wachstumsfaktorrezeptor als auch an einen Integrin-Rezeptor zu binden, die durch eine Zelle exprimiert werden, um dadurch die Migration und/oder Proliferation der Zelle zu induzieren, zu verstärken oder anderweitig zu fördern.

15. Isolierter Proteinkomplex nach einem der Ansprüche 1 bis 8 zur Verwendung für die Wund- oder Brandwunden-Heilung bei einem Tier, wobei vorzugsweise das Tier ein Mensch ist.

## Revendications

1. Complexe protéique isolé sous la forme d'une protéine chimère synthétique, comprenant une séquence d'acides aminés :
(i) d'un facteur de croissance ou d'au moins un domaine dudit facteur de croissance qui est capable de se lier à un récepteur de facteur de croissance apparenté ; et
(ii) d'un ou plusieurs domaines de type III de fibronectine (FN), dans lequel les un ou plusieurs domaines de type III de FN sont constitués des acides aminés 1266 à 1536 de la séquence de FN représentée dans SEQ ID NO : 1, ou des acides aminés 1447 à 1536 de la séquence de FN représentée dans SEQ ID NO : 1, ou des acides aminés 1173 à 1536 de la séquence de FN représentée dans SEQ ID NO : 1, lesquels un ou plusieurs domaines de type III de FN comprennent un domaine de liaison à l'intégrine.

2. Complexe protéique isolé selon la revendication 1, dans lequel ledit facteur de croissance est choisi parmi le facteur de croissance insulinomimétique de type I (IGF-I), le facteur de croissance insulinomimétique de type II (IGF-II), le facteur de croissance épidermique (EGF), le facteur de croissance basique des fibroblastes (bFGF), et le facteur de croissance des kératinocytes (KGF).

3. Complexe protéique isolé selon la revendication 1 ou la revendication 2, dans lequel le domaine de liaison à l'intégrine est un domaine de liaison à l'intégrine α₁ ou un domaine de liaison à l'intégrine α₄.

4. Complexe protéique isolé selon l'une quelconque des revendications 1 à 3, qui ne comprend pas une séquence d'acides aminés d'IGFBP.

5. Complexe protéique isolé selon l'une quelconque des revendications précédentes, comprenant en outre un fragment supplémentaire de FN.

6. Complexe protéique isolé selon la revendication 5, dans lequel le fragment supplémentaire de FN comprend les acides aminés 50 à 273 de la séquence de FN représentée dans SEQ ID NO : 1 ou les acides aminés 184 à 273 de la séquence de FN représentée dans SEQ ID NO : 1.

7. Complexe protéique isolé selon l'une quelconque des revendications précédentes, comprenant en outre au moins une séquence de lieur.

8. Complexe protéique isolé selon la revendication 7, dans lequel la séquence de lieur comprend un site de clivage de protéase, et/ou est choisie dans le groupe constitué par :
(i) Gly₄ Ser (SEQ ID NO : 7) ;
(ii) Gly₄ Ser₃ (SEQ ID NO : 8) ;
(iii) (Gly₄ Ser)₃ (SEQ ID NO : 9) ;
(iv) (Gly₄ Ser)₄ (SEQ ID NO : 10) ;
(v) Leu Ile Lys Met Lys Pro (SEQ ID NO : 11) ; et
(vi) Gln Pro Gln Gly Leu Ala Lys (SEQ ID NO : 12).

9. Acide nucléique isolé codant pour le complexe protéique isolé selon l'une quelconque des revendications précédentes.

10. Construction génétique, comprenant l'acide nucléique isolé selon la revendication 9 lié de manière fonctionnelle à une ou plusieurs séquences nucléotidiques de régulation dans un vecteur, dans laquelle de préférence la construction est une construction d'expression, et l'acide nucléique isolé est lié de manière fonctionnelle à un promoteur.

11. Cellule hôte comprenant la construction génétique selon la revendication 9 ou la revendication 10.

12. Composition pharmaceutique, comprenant le complexe protéique isolé selon l'une quelconque des revendications 1 à 8 et un support, diluant ou excipient pharmaceutiquement acceptable.

13. Implant, structure de maintien ou prothèse chirurgical imprégné, revêtu ou comprenant autrement le complexe protéique isolé selon l'une quelconque des revendications 1 à 8, ou un pansement pour plaie ou brûlure, comprenant le complexe protéique isolé selon l'une quelconque des revendications 1 à 8.

14. Procédé *in vitro* pour favoriser une migration et/ou une prolifération cellulaires, comprenant l'étape d'utilisation du complexe protéique isolé selon l'une quelconque des revendications 1 à 8 pour lier à la fois un récepteur de facteur de croissance et un récepteur d'intégrine exprimés par une cellule pour ainsi induire, augmenter ou favoriser d'une autre manière la migration et/ou la prolifération de ladite cellule.

15. Complexe protéique isolé selon l'une quelconque des revendications 1 à 8 pour son utilisation dans la guérison des plaies ou des brûlures chez un animal, dans lequel de préférence l'animal est un humain.
